# EUROPEAN PATENT SPECIFICATION

(11) **EP 4 005 477 B1**
(45) Date of publication and mention of the grant of the patent: **18.02.2026**
(21) Application number: 21196289.9
(22) Date of filing: 13.09.2021
(51) Int. Cl.: A61B 5/1455, A61B 5/00

(54) **BIOLOGICAL INFORMATION MEASURING APPARATUS AND ELECTRONIC DEVICE INCLUDING THE SAME**
GERÄT ZUR MESSUNG BIOLOGISCHER INFORMATIONEN UND ELEKTRONISCHE VORRICHTUNG DAMIT
APPAREIL DE MESURE D'INFORMATIONS BIOLOGIQUES ET DISPOSITIF ÉLECTRONIQUE LE COMPRENANT

(30) Priority: 30.11.2020 KR 20200164095; 05.03.2021 KR 20210029425
(43) Date of publication of application: 01.06.2022
(73) Proprietor: Samsung Electronics Co., Ltd., Suwon-si, Gyeonggi-do 16677 (KR)
(72) Inventor: Kwon, Yong Joo, Gyeonggi-do (KR); Kim, Kyoung Jun, Gyeonggi-do (KR); Lee, Joon Hyung, Gyeonggi-do (KR); Lee, Yeol Ho, Gyeonggi-do (KR); Choi, Seol Young, Gyeonggi-do (KR)
(74) Representative: Grünecker Patent- und Rechtsanwälte PartG mbB

(56) References cited:
- WO-A1-2020/075002
- TW-B- I 687 852
- US-A1- 2012 188 218
- US-A1- 2020 091 253
- CHE-I LIN ET AL: "A four transistor CMOS active pixel sensor with high dynamic range operation", ADVANCED SYSTEM INTEGRATED CIRCUITS 2004. PROCEEDINGS OF 2004 IEEE ASI A-PACIFIC CONFERENCE ON FUKUOKA, JAPAN 4-5 AUG. 2004, PISCATAWAY, NJ, USA,IEEE, US, 4 August 2004 (2004-08-04), pages 124 - 127, XP010733845, ISBN: 978-0-7803-8637-2, DOI: 10.1109/APASIC.2004.1349425

## Description

### BACKGROUND

### 1. Field

The present disclosure relates to a technology for measuring biological information, such as blood pressure, using a light-emitting pixel array and a light-receiving pixel array.

### 2. Description of Related Art

As medical science has progressed and average life expectancy has increased, an interest in health care has increased. Also, interest in medical equipment/devices has increased to extend not only to large-sized medical devices that can be utilized by hospitals and inspection agencies but also to middle-sized or small-sized medical devices and health-care devices that may be kept at home or carried by individuals. Medical devices for measuring biological information may include invasive devices and non-invasive devices. Using a non-invasive device, biological information may be detected in a relatively simple manner without causing pain to a subject, but the accuracy of the measurement result is low, and thus various studies to overcome such a drawback is being conducted.
US 2020/091253 A1 discloses an array substrate, a display panel and an operating method thereof. The array substrate includes a base substrate and an imaging array disposed on the substrate. The imaging array includes a photoelectric detection circuit and a temperature detection circuit. The photoelectric detection circuit includes a photosensitive sensor configured to detect light for imaging. The temperature detection circuit includes a temperature sensitive sensor configured to detect temperature. The imaging array is used for detecting light for imaging, so as to cooperate with other components arranged separately to realize functions of fingerprint recognition, iris recognition, face recognition, etc. When the array substrate is an OLED array substrate, the display pixel circuit may be any suitable pixel circuit such as 2T1C, 4T1C, 4T2C, etc.
WO 2020/075002 A1 discloses an imaging device that includes a substrate, a pixel array, and an adhesive layer. The substrate is flexible, the pixel array is positioned on a first surface of the substrate, and the adhesive layer is positioned on a second surface of the substrate opposite to the first surface. The pixel array has a light-receiving elements and light-emitting elements. The light-receiving elements have a function for detecting infrared light and each have a first pixel electrode, an active layer and a common electrode.
TW I 687 852 B discloses an electronic device cooperated with a human tissue and includes a body. The body has a sensing area that comprises a plurality of sensing units. Each sensing unit comprises a driving line, a signal line, a signal read-out line, a light-emitting element and an optical sensing element respectively. The light-emitting element is electrically connected with the driving line and the signal line respectively.
Che-I Lin et al: "A four transistor CMOS active pixel sensor with high dynamic range operation", Advanced System Integrated Circuits 2004, discloses CMOS APS using standard CMOS logic technology to allow high dynamic range operation. The cell is constructed by incorporating one additional transistor to the conventional three transistor APS. The cell offers flexible nonlinear transfer characteristics, which can be designed by modifying the operational timing diagram.
US 2012/0188218 Al discloses a memory-type liquid crystal display device includes a liquid crystal panel including memory circuits, and conducts a refresh operation more than once during a display holding period after rewriting of a screen. The memory-type liquid crystal panel includes a gate line, a source line, a transfer line, a refresh line, a retention capacitor line, a main transistor whose gate terminal is connected to the gate line, a pixel including a pixel electrode and a counter electrode, and a memory circuit for the pixel. The memory circuit includes a transfer transistor whose gate terminal is connected to the transfer line, a refresh transistor whose gate terminal is connected to the refresh line, a memory electrode, and a relay transistor whose gate terminal is connected to the memory electrode.

### SUMMARY

It is therefore the object of the present invention to provide an improved biological information measuring apparatus, an electronic device comprising a corresponding biological information measuring apparatus, and a corresponding method of operating a biological information measuring apparatus.

This object is solved by the subject matter of the independent claims.

Preferred embodiments are defined by the dependent claims.

This summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

In one general aspect, there is provided a biological information measuring apparatus including a light-emitting pixel array including a plurality of light-emitting pixel rows each including a plurality of light-emitting pixels, a light-receiving pixel array including a plurality of light-receiving pixel rows each including a plurality of light-receiving pixels and configured to sample light that is emitted from the light-emitting pixel array and reflected by an object, and a bio-signal processor configured to measure biological information using data sampled by the light-receiving pixel array, wherein each of the light-emitting pixels includes a storage capacitor configured to store an n^{th} light-emitting pixel signal and a memory capacitor configured to store an (n+1)^{th} light-emitting pixel signal.

**In** another general aspect, there is provided a biological information measuring apparatus including a light-emitting pixel array including a plurality of light-emitting pixel rows each including a plurality of light-emitting pixels, a light-receiving pixel array including a plurality of light-receiving pixel rows each including a plurality of light-receiving pixels and configured to sample light that is emitted from the light-emitting pixel array and reflected by an object, and a bio-signal processor configured to measure biological information using data sampled by the light-receiving pixel array, wherein the light-emitting pixel array includes a refresh line for simultaneously updating pixel signals of at least two or more light-emitting pixels that are arranged in different pixel rows.

In still another general aspect, there is provided an electronic device including a biological information measuring apparatus, a processor configured to control an operation of the biological information measuring apparatus, and a sound output device or display device configured to output information measured by the biological information measuring apparatus, wherein the biological information measuring apparatus includes a light-emitting pixel array including a plurality of light-emitting pixel rows each including a plurality of light-emitting pixels, a light-receiving pixel array including a plurality of light-receiving pixel rows each including a plurality of light-receiving pixels and configured to sample light that is emitted from the light-emitting pixel array and reflected by an object, and a bio-signal processor configured to measure biological information using data sampled by the light-receiving pixel array, wherein each of the light-emitting pixels includes a storage capacitor configured to store an n^{th} light-emitting pixel signal and a memory capacitor configured to store an (n+1)^{th} light-emitting pixel signal.

Other features and aspects will be apparent from the following detailed description, the drawings, and the claims.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a block diagram illustrating a biological information measuring apparatus according to an example embodiment;
FIG. 2A is a diagram illustrating an example in which a light-emitting pixel of FIG. 1 has a 4T-2C structure;
FIG. 2B is a diagram illustrating an example of a 2T-1C pixel driving circuit applied to a general display device;
FIG. 3 is a diagram for describing a refresh operation of a light-emitting pixel array of FIG. 1;
FIG. 4 is a diagram illustrating an example of a light-receiving pixel driving circuit in which a light-receiving pixel of FIG. 1 has a 4T structure;
FIG. 5 illustrates a combined pixel circuit in which a light-emitting pixel and a light-receiving pixel are combined;
FIG. 6A is a diagram illustrating a pixel array including infrared and green light-emitting pixels;
FIG. 6B is a diagram illustrating a state in which some infrared light-emitting pixels are turned on;
FIG. 6C is a diagram illustrating a state in which some green light-emitting pixels are turned on;
FIG. 6D is a diagram illustrating a state in which some green light-emitting pixels and some infrared emitting pixels are turned on;
FIG. 7A is a diagram illustrating a first photoplethysmogram (PPG) signal for light of a first wavelength;
FIG. 7B is a diagram illustrating a second PPG signal for light of a second wavelength;
FIG. 8 is a diagram for describing the principle of generating an element waveform included in a unit waveform of a PPG signal;
FIG. 9 is a diagram for describing an element waveform included in a unit waveform of a PPG signal;
FIG. 10 is a block diagram illustrating an electronic device including a biological information measuring apparatus;
FIG. 11 is a diagram illustrating an embodiment of a watch-type electronic device including a biological information measuring apparatus;
FIG. 12 is a diagram illustrating an embodiment of a mobile-type electronic device including a biological information measuring apparatus; and
FIG. 13 is a diagram illustrating an embodiment of an ear-wearable electronic device including a biological information measuring apparatus.

Throughout the drawings and the detailed description, unless otherwise described, the same drawing reference numerals will be understood to refer to the same elements, features, and structures. The relative size and depiction of these elements, features, and structures may be exaggerated for clarity, illustration, and convenience.

### DETAILED DESCRIPTION

Details of example embodiments are provided in the following detailed description with reference to the accompanying drawings. The disclosure may be understood more readily by reference to the following detailed description of example embodiments and the accompanying drawings. The disclosure may, however, be embodied in many different forms and should not be construed as being limited to the embodiments set forth herein. Rather, these embodiments are provided so that the disclosure will be thorough and complete and will fully convey the concept of the present disclosure to those skilled in the art, and the disclosure will only be defined by the appended claims. Like reference numerals refer to like elements throughout the specification.

It will be understood that, although the terms "first," "second," etc. may be used herein to describe various elements, these elements should not be limited by these terms. These terms are only used to distinguish one element from another. Also, the singular forms of terms are intended to include the plural forms of the terms as well, unless the context clearly indicates otherwise. In the specification, unless explicitly described to the contrary, the word "comprise," and variations such as "comprises" or "comprising," will be understood to imply the inclusion of stated elements but not the exclusion of any other elements. Terms such as "unit" and "module" denote units that process at least one function or operation, and they may be implemented by using hardware, software, or a combination of hardware and software.

The embodiments described below relate to the technical field of a biological information measuring apparatus (e.g., a blood pressure measuring apparatus), and detailed descriptions of matters widely known to those of ordinary skill in the relevant technical field will be omitted.

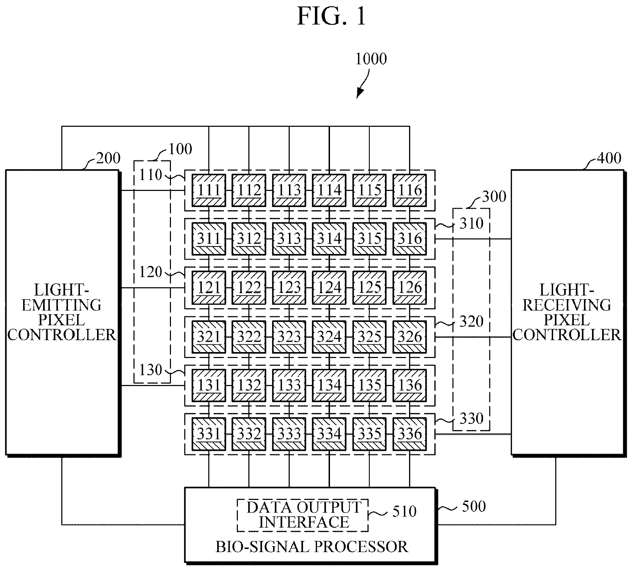

FIG. 1 is a block diagram illustrating a biological information measuring apparatus according to an example embodiment.

The biological information may include biological and medical information that may be obtained from an object OBJ to be measured, and may be, for example, a blood pressure, a blood sugar, body fat, a blood oxygen saturation, a blood vessel elasticity, a blood flow rate, or arterial stiffness. The object may be a part of a human body from which biological information is easily measured such as, for example, a region of a wrist adjacent to the radial artery or an upper area of the wrist through which capillary blood or venous blood passes, or a distal body portion, such as a finger, a toe, or the like, which has a high density of blood vessels.

Referring to FIG. 1, the biological information measuring apparatus 1000 may include a light-emitting pixel array 100 for emitting light to an object OBJ, a light-emitting pixel controller 200 for driving the light-emitting pixel array 100, a light-receiving pixel array 300 for detecting light returned after being scattered, reflected, or transmitted through the object OBJ, a light-receiving pixel controller 400 for driving the light-receiving pixel array 300, and a bio-signal processor 500 that measures biological information from a signal detected by the light-receiving pixel array 300.

The light-emitting pixel array 100 may be an array of light-emitting pixels that emit light to the object OBJ, and may include a plurality of light-emitting pixels 111, 121, etc. arranged in a two-dimensional manner. The light-emitting pixel array 100 may include a plurality of light-emitting pixel rows 110, 120, and 130 that include a plurality of light-emitting pixels 111, 121, etc. FIG. 1 illustrates the light-emitting pixel array 100 including three light-emitting pixel rows 110, 120, and 130 including a plurality of light-emitting pixels 111, 121, etc. The first to third light-emitting pixel rows 110, 120, and 130 of FIG. 1 may each include six light-emitting pixels 111, 121, etc., and for example, the first light-emitting pixel row 110 may include a 1-1 light-emitting pixel 111 to 1-6 light-emitting pixel 116.

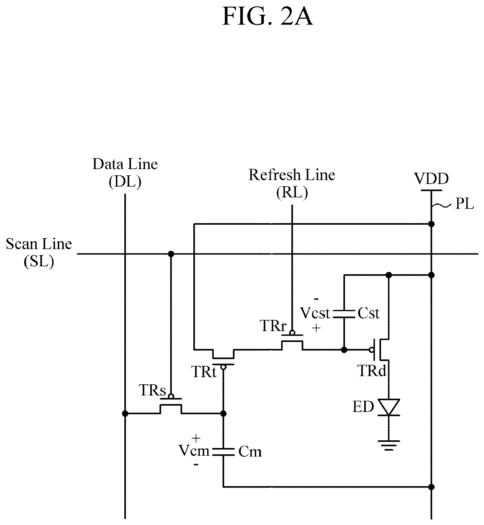

The light-emitting pixels 111, 121, etc. may each include a light-emitting element and a light-emitting pixel circuit for driving the light-emitting element. The light-emitting element included in each of the light-emitting pixels 111, 121, etc., may be a light source, such as a light-emitting diode (LED), a laser diode (LD), an organic light-emitting diode (OLED), or the like, and the structure of the light-emitting pixel circuit will be described below with reference to FIG. 2A.

The light-emitting pixel array 100 may include the light-emitting pixels 111, 121, etc., which emit light of two or more different wavelengths that penetrate to different depths in the object OBJ. For example, a first, a third, and a fifth light-emitting pixels 111, 113, and 115 included in the first light-emitting pixel row 110 may emit light of a first wavelength, and the other pixels included in the first light-emitting pixel row 110, that is, a second, a fourth, and a sixth light-emitting pixels 112, 114, and 116 may emit light of a second wavelength. The light of the first wavelength may be long-wavelength light having a high penetration depth into the object OBJ such as, for example, light in an infrared wavelength range (e.g., 750 nm to 2500 nm). The light of a second wavelength may be a short-wavelength light that has a smaller penetration depth into the object OBJ than the light of the first wavelength such as, for example, light in a green wavelength range (e.g., 500 nm to 565 nm).

The light-emitting pixel array 100 may include various combinations of light-emitting pixels 111, 121, etc., in addition to the structure illustrated above, and may be configured such that one light-emitting pixel row 110, 120, or 130 includes light-emitting pixels that emit three or four different wavelengths. In another example, each pixel row may be configured to emit light of a different wavelength. In this case, the first light-emitting pixel row 110 may emit infrared light, the second pixel row 120 may emit green light, and the third pixel row 130 may emit blue light.

The light-emitting pixel controller 200 may supply power to the light-emitting pixel array 100, and may generate and transmit signals for driving the light-emitting pixel array 100, such as a scan line signal, a data line signal, and a refresh signal. The light-emitting pixel controller 200 may independently control the on/off operation and light emission amount of each of the light-emitting pixels 111, 121, etc.

The light-receiving pixel array 300 is an array of light-receiving pixels that detect light returning from the object OBJ and generate electrical signals, and may include a plurality of light-receiving pixels 311, 312, etc., which are arranged in a two-dimensional manner. The light-receiving pixel array 300 may include a plurality of light-receiving pixel rows 310, 320, and 330 including a plurality of light-receiving pixels 311, 321, etc. FIG. 1 illustrates a light-receiving pixel array 300 that includes three light-receiving pixel rows 310, 320, and 330 including a plurality of light-receiving pixels 311, 321, etc. A first to third light-receiving pixel rows 310, 320, and 330 may each include six light-receiving pixels 311, 321, etc. For example, the first light-receiving pixel row 310 may include a 1-1 light-receiving pixel 311 to 1-6 light-receiving pixel 316.

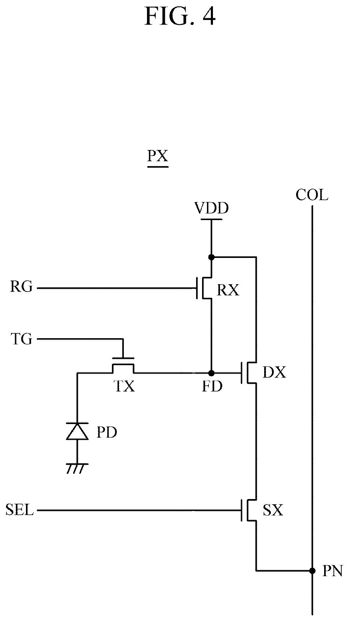

The light-receiving pixels 311, 321, etc., may each include a photoelectric conversion element for converting light into an electric signal and a light-receiving pixel circuit for driving the photoelectric conversion element. The photoelectric conversion element included in each of the light-receiving pixels 311, 321, etc., may be implemented as a photo diode, a photo transistor, a photogate, a pinned photo diode, or the like. A color filter for selectively transmitting or removing light of a specific wavelength may be disposed on an upper portion of each of the light-receiving pixels 311, 321, etc. The structure of the light-receiving pixel circuit will be described below with reference to FIG. 4.

One or a plurality of light-receiving pixel rows 310, 320, and 330 may be disposed between the light-emitting pixel rows 110, 120, and 130. For example, as shown in FIG. 1, the first light-receiving pixel row 310 may be disposed between the first to second light-emitting pixel rows 110 and 120. Although FIG. 1 illustrates that one light-receiving pixel row is disposed between the light-emitting pixel rows 110, 120, and 130, two or more light-receiving pixel rows such as, for example, two to two thousand light-receiving pixel-rows, may be disposed between the light-emitting pixel rows 110, 120, and 130.

In addition, FIG. 1 illustrates an example in which one light-emitting pixel row is disposed between the light-receiving pixel rows 310, 320, and 330, but two or more such as, for example, two to two thousand light-emitting pixel rows may be disposed between the light-receiving pixel rows 310, 320, and 330.

The light-receiving pixel controller 400 may supply power to the light-receiving pixel array 300, and may generate and transmit light-receiving pixel driving signals, such as a timing signal, a transmission signal TG, a reset signal RG, a selection signal SEL, and the like, that for driving the light-receiving pixel array 300. The light-receiving pixel controller 400 may drive the light-receiving pixel array 300 using a rolling shutter method by which the light-receiving pixel rows 310, 320, and 330 are sequentially exposed to light and read out, or using a global shutter method by which the light-receiving pixel rows 310, 320, and 330 are simultaneously exposed to light and read-out.

The bio-signal processor 500 may measure biological information using data output by the light-receiving pixel array 300. Output data of the light-receiving pixel array 300 received by the bio-signal processor 500 may be data related to the intensity of light detected by the light-receiving pixels 311, 321, etc., and the bio-signal processor 500 may analyze/extract biological information by analyzing the received data.

The bio-signal processor 500 may include components for processing light sensing data in a conventional image sensor, such as a complementary metal-oxide-semiconductor (CMOS) image sensor. For example, the bio-signal processor 500 may include a data output interface 510 which receives light-receiving pixel signals in an analog form from the light-receiving pixels 311, 321, etc., converts the received signals to digital signals, temporarily stores the digital signals, amplifies the digital signals, and outputs the amplified digital signals to the outside. Specifically, the data output interface 510 may include an analog-to-digital converter (ADC), may compare the magnitude of correlated double sampled analog signals of the light-receiving pixels 311, 321, etc., with the magnitude of a ramp signal to generate comparison signals corresponding to a difference in magnitude of each signal, and may convert the analog signals to digital signals by counting the comparison signals. The data output interface 510 may include a memory for storing the digital signals and a buffer including an amplifier for sensing and amplifying the digital signals.

**In** addition, the bio-signal processor 500 may generate a timing signal and a driving signal to enable the light-emitting pixel array 100 and the light-receiving pixel array 300 to interwork with each other and transmit the generated timing signal and driving signal to the light-emitting pixel controller 200 and the light-receiving pixel controller 400. For example, the bio-signal processor 500 may generate timing signals based on a master clock signal received from a processor (e.g., a central processing unit (CPU), and the like) and/or a clock signal generated by a separate clock generator and transmit the generated timing signals to the light-emitting pixel controller 200 and the light-receiving pixel controller 400. The timing signal may be a pixel clock that is used as a reference for a series of operations, such as a light-emission operation of the light-emitting pixel array 100, a photoelectric conversion operation of the light-receiving pixel array 300, an analog-to-digital conversion operation of the data output interface 510, and the like, that are performed in the biological information measuring apparatus 1000.

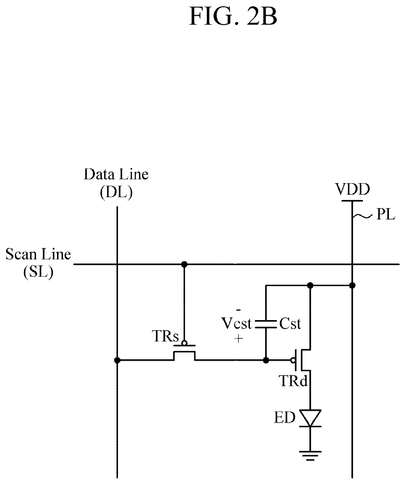

FIG. 2A is a diagram illustrating an example in which a light-emitting pixel of FIG. 1 has a 4T-2C structure, and FIG. 2B is a diagram illustrating an example of a 2T-1C pixel driving circuit applied to a general display device.

Referring to FIG. 2A, each of the light-emitting pixels 111, 121, etc., may include a light-emitting element ED and a pixel circuit. The pixel circuit may include a switching transistor TRs, a transfer transistor TRt, a refresh transistor TRr, a driving transistor TRd, a memory capacitor Cm, and a storage capacitor Cst.

The switching transistor TRs may be connected to a scan line SL and a data line DL, and transmit a data signal, which is input through the data line DL, to the memory according to a scan signal input through the scan line SL.

The memory capacitor Cm may be connected to the switching transistor TRs, the transfer transistor TRt, and a driving voltage line PL. The memory capacitor Cm may store a voltage Vcm corresponding to a difference between a voltage of the data line DL and a driving voltage VDD through the switching transistor TRs. A memory capacitor voltage Vcm may be a value corresponding to a data signal input through the data line DL.

The transfer transistor TRt may be connected to the driving voltage line PL and the storage capacitor Cst, and may provide a voltage corresponding to the memory capacitor voltage Vcm to the refresh transistor TRr.

The refresh transistor TRr may be connected to the refresh line RL, the transfer transistor TRt, and the storage capactior Cst, and may provide a voltage transferred from the transfer transistor TRt to the storage capacitor Cst according to a refresh signal input through the refresh line RL. As described above, the voltage provided by the transfer transistor TRt is a value corresponding to the memory capacitor voltage Vcm, and thus the refresh transistor TRr may provide the memory capacitor voltage Vcm to the storage capacitor Cst.

The storage capacitor Cst may be connected to the refresh transistor TRr, the driving transistor TRd, and the driving voltage line PL, and may store a voltage Vcst corresponding to a difference between a voltage provided by the refresh transistor TRr and the driving voltage VDD. The voltage Vcst of the storage capacitor may be charged by the memory capacitor Cm. Since the voltage Vcst of the storage capacitor determines a magnitude of a voltage to be applied to the light-emitting element ED, that is, the on/off operation and light emission amount of the light-emitting element, the storage capacitor stores a light-emitting pixel signal.

The driving transistor TRd may be connected to the storage capacitor Cst and the driving voltage line PL, and may provide a driving current corresponding to the voltage stored in the storage capacitor Cst to the light-emitting element ED.

The light-emitting pixel driving circuit of FIG. 2A is different from a 2T-1C active matrix pixel driving circuit in that it includes the refresh line RL, the refresh transistor TRr, the transfer transistor TRt, and a memory capacitor Cm.

In a 2T-1C light-emitting pixel circuit of FIG. 2B, a signal transmitted to a pixel through the data line DL is directly stored in the storage capacitor Cst through the switching transistor TRs without passing through the memory capacitor Cm, whereas a signal of the data line DL of FIG. 2A is not directly stored in the storage capacitor Cst, but is stored in the memory capacitor Cm and then transmitted to the storage capacitor Cst in response to the signal of the refresh line RL.

In addition, the light-emitting pixel circuit of FIG. 2B is different from the light-emitting pixel circuit of FIG. 2A in that the storage capacitor voltage Vcst is refreshed by the signal of the scan line SL, whereas the storage capacitor voltage Vcst in the light-emitting pixel circuit of FIG. 2A is refreshed by a signal of the refresh line RL provided separately from the scan line RL. Accordingly, the light-emitting pixel rows in the light-emitting pixel array including the light-emitting pixel circuit of FIG. 2B are sequentially refreshed corresponding to a signal of the scan line SL, whereas all light-emitting pixels 111, 121, etc., that are included in the light-emitting pixel array of FIG. 1 including the light-emitting pixel circuit of FIG. 2A are simultaneously refreshed. In other words, in the light-emitting pixel array 100 of FIG. 1, the light-emitting pixels 111, 121, etc. included in different light-emitting pixel rows are simultaneously refreshed using the refresh line RL.

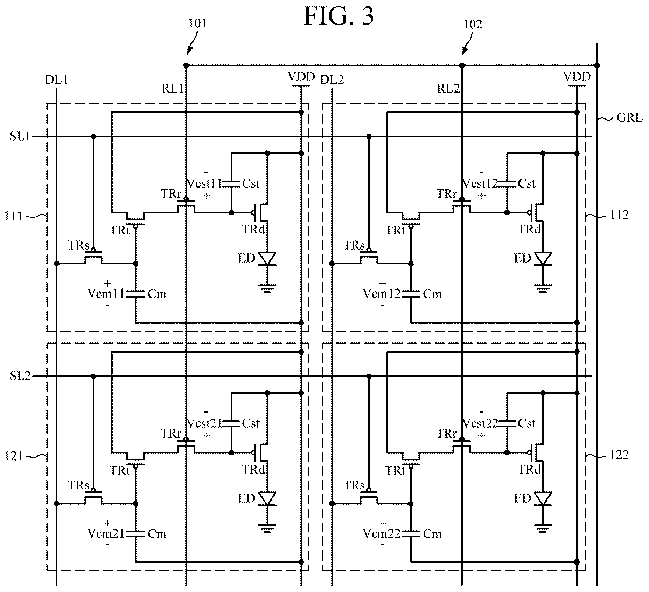

FIG. 3 is a diagram for describing a refresh operation of a light-emitting pixel array of FIG. 1.

FIG. 3 illustrates a pixel circuit of four adjacent light-emitting pixels 111, 112, 121, and 122 among the light-emitting pixels of FIG. 1. The light-emitting pixels 111, 112, 121, and 122 shown in FIG. 3 may include a 4T-2C structure as described above with reference to FIG. 2A.

Referring to FIG. 3, the 1-1 and 2-1 light-emitting pixels 111 and 121 included in the first light-emitting pixel row 101 may share a first refresh line RL1, and the 1-2 and 2-2 light-emitting pixels 112 and 122 included in the second light-emitting pixel row may share a second refresh line RL2. The first and second refresh lines RL1 and RL2 may be connected to a common refresh line GRL. That is, when the light-emitting pixel controller 200 applies a refresh signal through the common refresh line GRL, all light-emitting pixels 111, 112, 121, and 122 may be simultaneously refreshed.

Table 1 below shows how voltages of the storage capacitor Cst and the memory capacitor Cm change by the refresh signal.

**Table 1**

| Before application of refresh signal (t=0) | | After application of refresh signal (t=1) | |
|---|---|---|---|
| Vcst11 = V11t0 | Vcm11 = V11t1 | Vcst11 = V11t1 | Vcm11 = V11t1 → V11t2 |
| Vcst12 = V12t0 | Vcm12 = V12t1 | Vcst12 = V12t1 | Vcm12 = V12t1 → V12t2 |
| Vcst21 = V21t0 | Vcm21 = V21t1 | Vcst21 = V21t1 | Vcm21 = V21t1 → V21t2 |
| Vcst22 = V22t0 | Vcm22 = V22t1 | Vcst22 = V22t1 | Vcm22 = V22t1 → V22t2 |

Describing the state of the light-emitting pixels 111, 112, 121, 122 of FIG. 3 before application of the refresh signal (t=0) with reference to Table 1, storage capacitor voltages Vcst11, Vcst12, Vcst21, and Vcst22 are V11t0, V12t0, V21t0, and V22t0, respectively, memory capacitor voltages Vcm11, Vcm12, Vcm21, and Vcm22 are charged to V11t1, V12t1, V21t1, and V22t1, respectively, and each light-emitting element ED is driven by a current corresponding to each of the storage capacitor voltages Vcst11,Vcst12, Vcst21, and Vcst22.

When the refresh signal is applied, refresh transistors TRr included in the light-emitting pixels 111, 112, 121, and 122 are simultaneously turned on (switch close) so that the storage capacitor voltages Vcst11, Vcst12, Vcst21, and Vcst22 are updated to the memory capacitor voltages Vcm11, Vcm12, Vcm21, and Vcm22, respectively. That is, the storage capacitor voltages Vcst11, Vcst12, Vcst21, and Vcst22 are, respectively, changed from V11t0, V12t0, V21t0, and V22t0 to V11t1, V12t1, V21t1, and V22t1, which are the memory capacitor voltages Vcm11, Vcm12, Vcm21, and Vcm22.

Upon the completion of the refresh operation, the refresh signal may be blocked to turn off the refresh transistors TRr (switch open), and the memory capacitor voltages Vcm11, Vcm12, Vcm21, and Vcm22 may be updated. The memory capacitor voltages Vcm11, Vcm12, Vcm21, and Vcm22 may proceed sequentially for each pixel row through a data line DL1 and a scan line SL1. Referring to Table 1, the memory capacitor voltages Vcm11, Vcm12, Vcm21, and Vcm22 may be, respectively, updated from V11t1, V12t1, V21t1, and V22t1 to V11t2, V12t2, V21t2, and V22t2. The storage capacitor voltages Vcst11, Vcst12, Vcst21, and Vcst22 may be unchanged while the memory capacitor voltages Vcm11, Vcm12, Vcm21, and Vcm22 are being updated, and each light-emitting element ED may be driven by a current corresponding to each of the storage capacitor voltages Vcst11, Vcst12, Vcst21, and Vcst22, that is, V11t1, V12t1, V21t1, and V22t1.

Subsequently, when the refresh signal is applied again, the storage capacitor voltages Vcst11, Vcst12, Vcst21, and Vcst22 may be respectively updated from V11t1, V12t1, V21t1, and V22t1 to V11t2, V12t2, V21t2, V22t2, and simultaneous updating of the storage capacitors Cst and sequential updating of the memory capacitors Cm may be repeatedly performed.

In summary, since the storage capacitor Cst stores the current light-emitting pixel signal applied to the light-emitting element ED, the storage capacitor voltage Vcst may be referred to as an n^{th} light-emitting pixel signal, and since the memory capacitor Cm stores the next light-emitting pixel signal, the memory capacitor voltage Vcm may be referred to as an (n+1)^{th} light-emitting pixel signal.

FIG. 4 illustrates an example in which the light-receiving pixel of FIG. 1 has a 4T structure.

Referring to FIG. 4, each of the light-receiving pixels 311, 321, etc. may include a photo diode PD and a pixel circuit. The pixel circuit may include a floating diffusion FD, a transfer transistor TX, a reset transistor RX, a driving transistor DX, and a selection transistor SX.

The floating diffusion FD is a connection part between the transfer transistor TX, the reset transistor RX, and the driving transistor DX, and may be a portion that accumulates charges photoelectrically converted by the photo diode PD and converts the charges into voltage. The transfer transistor TX may be turned on to transfer the charges photoelectrically converted by the photo diode PD to the floating diffusion FD. The reset transistor RX is turned on to make a voltage of the floating diffusion FD be a power voltage VDD, and removes the charged accumulated in the floating diffusion FD. The driving transistor DX may amplify a signal, that is, voltage, caused by the charges accumulated in the floating diffusion FD. The selection transistor SX may be turned on to output voltage, that is, a light-receiving pixel signal, amplified by the driving transistor DX to a column line COL. Among signals for controlling on/off of the transistors TX, RX, and SX, a signal for controlling the transfer transistor TX is referred to as a transfer signal TG, a signal for controlling the reset transistor RX is referred to as a reset signal RG, and a signal for controlling the selection transistor SX is referred to as a selection signal SEL.

A light-receiving pixel circuit of FIG. 4 may be used when the light-receiving pixel array 300 is operated by a rolling shutter method, and a light-receiving pixel circuit of a different structure may be applied when the light-receiving pixel array 300 is intended to be operated by a global shutter method. Since various structures for the light-receiving pixel circuit driven by the global shutter method are known, detailed descriptions thereof are omitted herein.

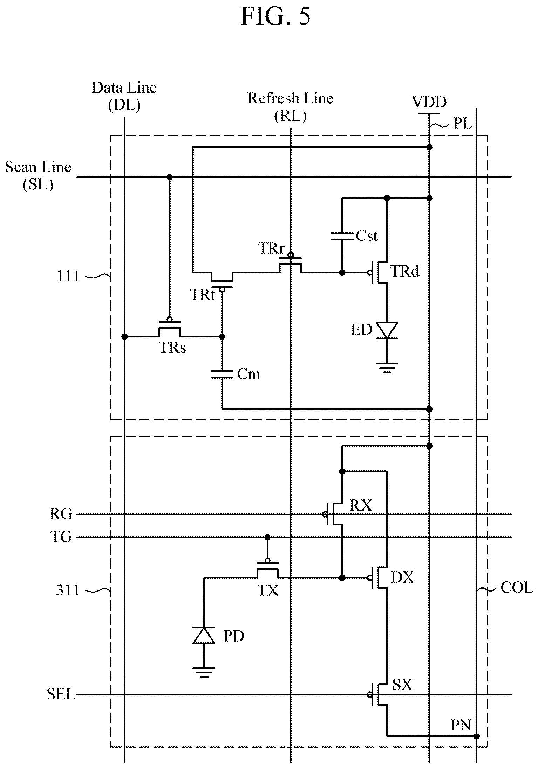

FIG. 5 illustrates a combined pixel circuit in which a light-emitting pixel and a light-receiving pixel are combined.

FIG. 5 illustrates a pixel circuit in which the 1-1 light-emitting pixel 111 of FIG. 1 and the 1-1 light-receiving pixel 311 adjacent to the 1-1 light-emitting pixel 111 are combined. The 1-1 light-emitting pixel 111 may include the pixel circuit of FIG. 2A, and the 1-1 light-receiving pixel 311 may include the pixel circuit of FIG. 4.

The 1-1 light-emitting pixel 111 and the 1-1 light-receiving pixel 311 of FIG. 5 may share a driving voltage line PL, for example, a driving transistor TRd of the 1-1 light-emitting pixel 111 and a reset transistor RX of the 1-1 light-receiving pixel 311 may be connected to the same driving voltage line PL.

The biological information measuring apparatus 1000 of FIG. 1 has the same number of light-emitting pixels 111, 112, etc., as the light-receiving pixels 311, 312, etc., but the number of light-emitting pixels 111, 112, etc., may be different from the number of light-receiving pixels 311, 312, etc. For example, the number of light-receiving pixels 311, 312, etc. may be greater than the number of light-emitting pixels 111, 112, etc.

The biological information measuring apparatus 1000 of FIG. 1 may include a structure in which all the light-emitting pixels 111, 112, etc., and all the light-receiving pixels 311, 312, etc. are combined as shown in FIG. 5, a structure in which only some light-emitting pixels 111, 112, etc., and some light-receiving pixels 311, 312, etc., are combined, and a structure in which all the light-emitting pixels 111, 112, etc., and all the light-receiving pixels 311, 312, etc., are not combined, that is, a structure in which the circuit of the light-emitting pixel array 100 and the circuit of the light-receiving pixel array 300 are separated from each other.

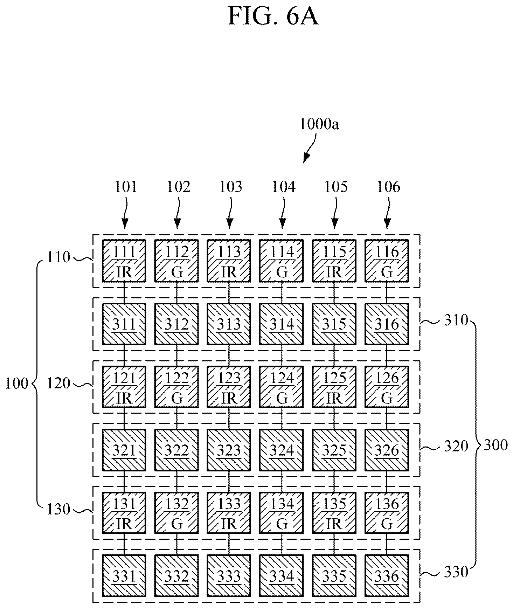

Hereinafter, an example of a method of measuring a photoplethysmogram (PPG) signal and a blood pressure among various types of biological information using the biological information measuring apparatus 1000 of FIG. 1 will be described with reference to FIGS. 6A to 9.

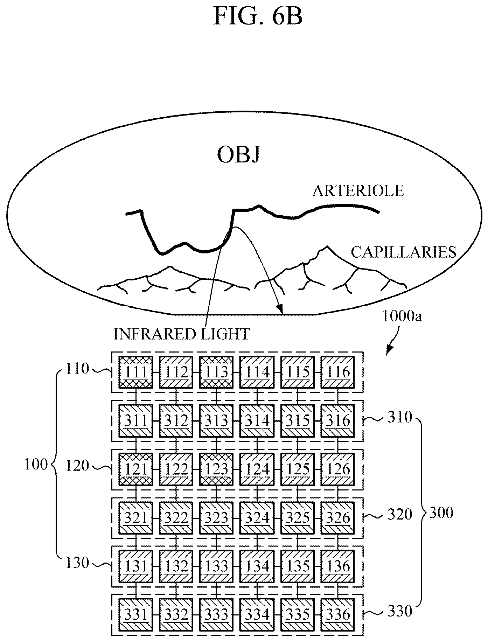

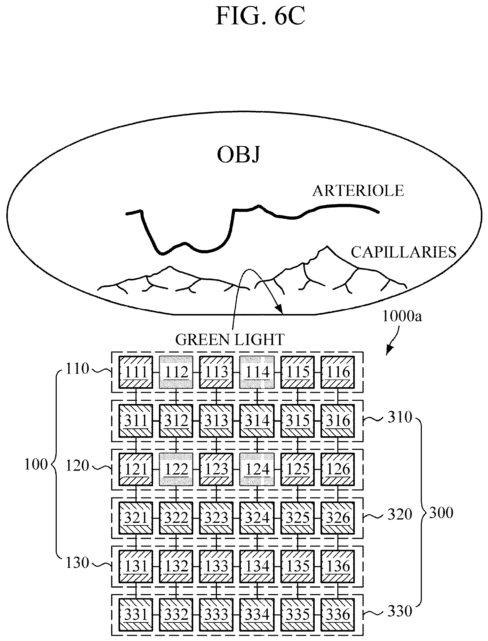

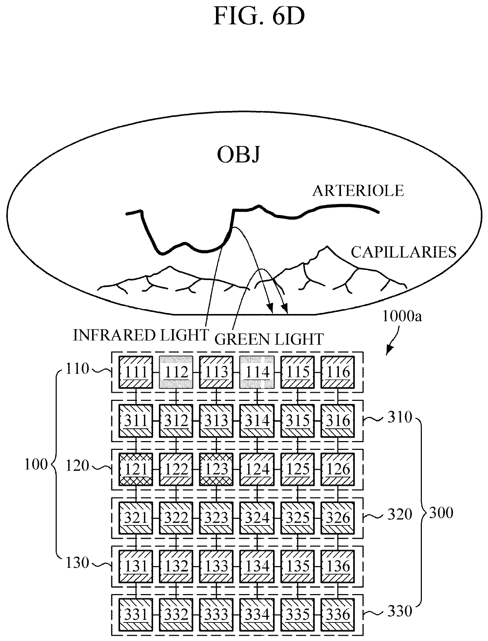

FIG. 6A is a diagram illustrating a pixel array including infrared and green light-emitting pixels, FIG. 6B is a diagram illustrating a state in which some infrared light-emitting pixels are turned on, FIG. 6C is a diagram illustrating a state in which some green light-emitting pixels are turned on, and FIG. 6D is a diagram illustrating a state in which some green light-emitting pixels and some infrared emitting pixels are turned on.

FIG. 6A illustrates an example in which the biological information measuring apparatus 1000 of FIG. 1 includes light-emitting pixels that emit light of two different wavelengths, wherein the light-emitting pixel controller 200, the light-receiving pixel controller 400, and the bio-signal processor 500 are omitted. The two different wavelengths may be, for example, infrared IR and green light G. The infrared emitting pixels and the green light-emitting pixels may be arranged in various ways. For example, as shown in FIG. 6A, the infrared emitting pixels and the green light-emitting pixels may be alternately arranged. Specifically, a biological information measuring apparatus 1000a of FIG. 6A may include infrared emitting pixels 111, 121, 131, etc., arranged in a first, third, and fifth light-emitting pixel rows, and green light-emitting pixels 112, 122, 132, etc., arranged in a second, fourth, and sixth light-emitting pixel rows 102, 104, and 106.

The light-emitting pixel controller 200 of FIG. 6A may independently control each of the light-emitting pixels 111, 112, etc., and may turn on or turn off all the light-emitting pixels 111, 112, etc., or may selectively turn on one or some light-emitting pixels as necessary. For example, , the light-emitting pixel controller 200 may selectively turn on only four infrared emitting pixels 111, 113, 121, and 123 among the light-emitting pixels 111, 112, etc., as shown in FIG. 6B, or may selectively turn on only four green light-emitting pixels 112, 114, 122, and 124 as shown in FIG. 6C.

The position of the light-emitting pixel to be turned on may be determined by taking into account the part of the object OBJ, such as a finger, a wrist, or the like, a position where the object OBJ is in contact with the biological information measuring apparatus 1000a, and the type of biological information, such as blood pressure, body fat, or the like.

As shown in FIG. 6B, when the infrared emitting pixels 111, 113, 121, and 123 are turned on to emit infrared light to the object OBJ, infrared light scattered, reflected, or transmitted through the object OBJ may be sampled in the light-receiving pixel array 300. Alternatively, as shown in FIG. 6C, when the green light-emitting pixels 112, 114, 122, and 124 are turned on to emit green light to the object OBJ, green light returning from the object OBJ may be sampled in the light-receiving pixel array 300.

Since the biological information measuring apparatus 1000a of FIG. 6A includes a memory capacitor Cm and a refresh line RL, light-emitting pixel signals applied to all the light-emitting pixels 111, 112, etc., may be simultaneously updated. For example, the state of the light-emitting pixel array 100 of FIG. 6A may be switched from a state in which the four infrared emitting pixels 111, 113, 121, and 123 of FIG. 6B are turned on to a state in which the four green light-emitting pixels 112, 114, 122, and 124 of FIG. 6C are turned on. If the light-emitting pixels 111, 112, etc. of FIG. 6A have a conventional 2T-1C structure as shown in FIG. 2B, the light-emitting pixel signals are not simultaneously updated for all the light-emitting pixels 111, 112, etc., but are sequentially updated with a time difference for each of the light-emitting pixel rows 110, 120, and 130. Thus, there may be a phase during which infrared light and green light are simultaneously emitted as shown in FIG. 6D. In other words, in the process of switching the state of the light-emitting pixel array 100 from the state shown in FIG. 6B to the state shown in FIG. 6C, before a pixel signal of the second light-emitting pixel row 120 is updated after a pixel signal of the first light-emitting pixel row 110 is updated, the light-emitting pixel array 100 may pass through a state in which infrared light and green light are simultaneously emitted. The signals which are sampled by the light-receiving pixel array 300 while the light-emitting pixel array 100 is simultaneously emitting light of two or more wavelengths are often noise signals that are difficult to use for bio-signal analysis, and thus it may be desirable that the light-emitting pixel array 100 does not simultaneously emit light of two or more wavelengths. As described above, since the light-emitting pixel array 100 of FIG. 6A includes the memory capacitor Cm and the refresh line RL, light of two or more wavelengths may not be undesirably emitted simultaneously, and the light-receiving pixel array 300 may continuously sample valid signals.

The biological information measured by the biological information measuring apparatus of FIG. 6A may be a PPG signal. The PPG signal is a signal obtained by emitting light of a specific wavelength band to the human body and detecting reacted light, and may be a signal indicating a ripple component generated due to heartbeat. The bio-signal processor 500 may obtain PPG signals for infrared light and green light by using the data sampled by the light-receiving pixel array 300.

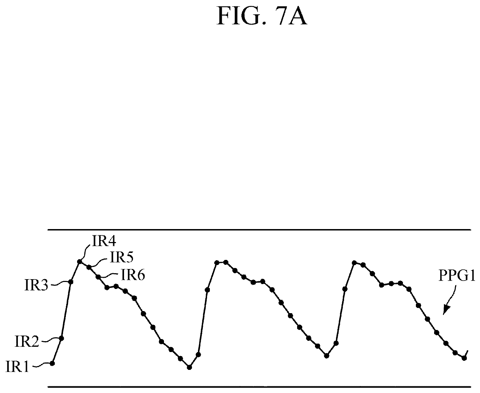

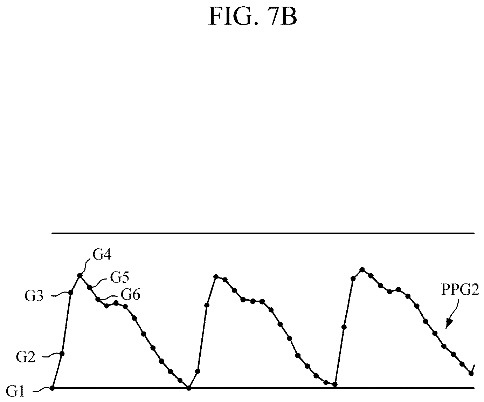

FIG. 7A shows an example of a first PPG signal PPG1 for infrared light obtained from the light-receiving pixel array 300, and FIG. 7B shows an example of a second PPG signal PPG2 for green light.

Referring to FIGS. 7A and 7B, signals IR1, G1, etc., that constitute the first and second PPG signals PPG1 and PPG2 may correspond to signals sampled by alternately repeating the turn-on state of the infrared emitting pixels 111, 113, 121, and 123 as shown in FIG. 6B and the turn-on state of the green light-emitting pixels 112, 114, 122, and 124 as shown in FIG. 6C. For example, IR1 of FIG. 7A may correspond to a first infrared signal sampled in the state of FIG. 6B in which the infrared emitting pixels are turned on, and IR6 may correspond to a sixth infrared signal sampled in the state of FIG. 6B in which the infrared emitting pixels are turned on. Similarly, G1 of FIG. 7B may correspond to a first green light signal sampled in the state of FIG. 6C in which the green light-emitting pixels are turned on, and G6 may correspond to a sixth green light signal sampled in the state of FIG. 6C in which the green light-emitting pixels are turned on. The infrared light signal and the green light signal may be alternately sampled, such as sampling the first infrared light signal IR1 and then sampling the first green light signal G1.

The light-receiving pixel signals sampled by the light-receiving pixel array 300 may include light-receiving pixel signals incoming from the plurality of light-receiving pixels 311, 312, etc., and the bio-signal processor 500 may obtain a PPG signal by processing the light-receiving pixel signals in various ways. For example, a PPG signal may be obtained by averaging the light-receiving pixel signals sampled by all the light-receiving pixels 311, 312, etc., by averaging the light-receiving pixel signals sampled by a specific light-receiving pixel row, for example, the second light-receiving pixel row 320, or by using only the signal sampled by a light-receiving pixel disposed at a specific position, for example, the 3-3 light-receiving pixel 333.

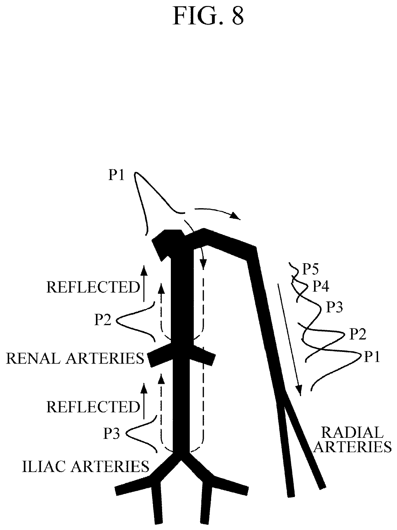

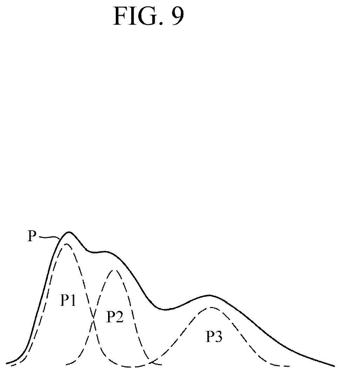

FIG. 8 is a diagram for describing the principle of generating a unit waveform constituting a PPG signal, and FIG. 9 is a diagram illustrating one unit waveform P constituting a PPG signal divided into a plurality of element waveforms.

Referring to FIGS. 8 and 9, the unit waveform P may be composed of a superposition of a propagation wave P1 starting from the heart toward the vascular bifurcations at the body distal ends, such as the iliac arteries, and reflection waves P2 and P3 returning from the distal ends or the vascular bifurcations. Specifically, element waveforms P1, P2, and P3 may include a propagation wave P1 caused by the contractile motion of the heart, a first reflection wave P2 mainly reflected from the renal arteries, and a second reflection wave P3 main reflected from the iliac arteries. The amplitude of the propagation wave P1 is highly related to the movement of the heart and the amplitude of the reflection waves P2 and P3 are highly related to the characteristics of blood vessels. In this way, the unit waveform P of the PPG signal is divided into the element waveforms P1, P2, and P3, and blood pressure may be measured by analyzing the intensity and duration of each of the element waveforms P1, P2, and P3, an interval between the element waveforms P1, P2, and P3, the ratio of the intensity, and the like. For example, blood pressure may be measured by analyzing a time interval and/or a ratio of the maximum intensity of peak points of the propagation wave P1 and the reflection waves P2 and P3. Specifically, the first PPG signal PPG1 of FIG. 7A and the second PPG signal PPG2 of FIG. 7B may be analyzed by dividing each of them into element waveforms P1, P2, and P3, and values obtained from the analysis may be averaged to measure blood pressure, or based on the second PPG signal PPG2 of FIG. 7B, information derived from the first PPG signal PPG1 of FIG. 7A may be used to correct a value obtained by analyzing the second PPG signal PPG2, thereby increasing the accuracy of blood pressure measurement.

In the embodiment of FIG. 6A, a method of measuring a PPG signal or blood pressure using green and infrared wavelength light has been described as an example. However, the biological information measuring apparatus 1000 of FIG. 1 may use light of other wavelengths, such as blue light, and may be used to measure other biological information, such as a blood sugar, body fat, a blood oxygen saturation, a blood vessel elasticity, a blood flow rate, and arterial stiffness, in addition to blood pressure.

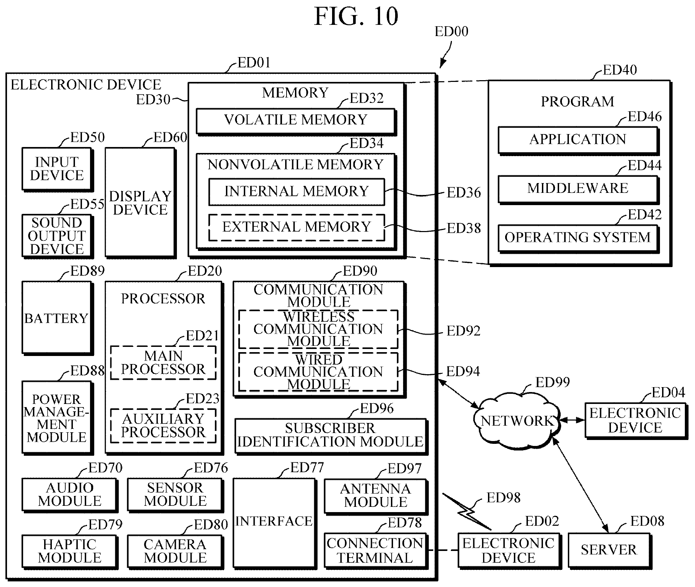

FIG. 10 is a block diagram illustrating an electronic device including a biological information measuring apparatus.

An electronic device ED01 may include a processor ED20, a memory ED30, an input device ED50, a sound output device ED55, a display device ED60, an audio module ED70, a sensor module ED76, an interface ED77, a haptic module ED79, a camera module ED80, a power management module ED88, a battery ED89, a communication module ED90, a subscriber identification module ED96, and/or an antenna module ED97. Some of these components (such as the display device ED60) may be omitted, or other components may be added. The biological information measuring apparatuses 1000 and 1000a described above with reference to FIGS. 1 and 6A may be implemented as integrated circuitry and mounted in the sensor module DE76 of the electronic device ED01 or may be distributed in different components of the electronic device ED01. For example, the light-emitting pixel array 100 and/or the light-receiving pixel array 300 may be included in the sensor module ED76, and the light-emitting pixel controller 200, the light-receiving pixel controller 400, and/or the bio-signal processor 500 may be included in the processor ED20.

The processor ED20 may execute software (e.g., program ED40, etc.) to control one or a plurality of other components (e.g., hardware, software components, etc.) of the electronic devices ED01 connected to the processor ED20, and may perform various data processing or operations. As part of data processing or operation, the processor ED20 may load commands and/or data received from other components (e.g., the sensor module ED76, the communication module ED90, etc.) into a volatile memory ED32, process the commands and/or data stored in the volatile memory ED32, and store result data in a nonvolatile memory ED34. The processor ED20 may generate a master clock for synchronizing operations of the components and provide the master clock to other components such as, for example, the light-emitting and light-receiving pixel controllers 200 and 400 and/or the bio-signal processor 500 of FIG. 1.

The processor ED20 may include a main processor ED21 (e.g., central processing unit, application processor, etc.) and an auxiliary processor ED23 (e.g., graphic processing unit, image signal processor, sensor hub processor, communication processor, etc.) that can be operated independently or together with the main processor ED21. The auxiliary processor ED23 may use less power than the main processor ED21 and may perform specialized functions. The auxiliary processor ED23 may control functions and/or states related to some components (e.g., the display device ED60, the sensor module ED76, the communication module ED90, etc.) among the components of the electronic device ED01 in substitute for the main processor ED21 while the main processor ED21 is in an inactive state (sleep state), or together with the main processor ED21 while the main processor ED21 is in an active state (application execution state). The auxiliary processor ED23 (e.g., an image signal processor, a communication processor, etc.) may be implemented as a part of other functionally related components (e.g., the camera module ED80, the communication module ED90, etc.).

The processor ED20 may transmit a control signal to the light-emitting and light-receiving pixel controllers 200 and 400 and/or the bio-signal processor 500 of the biological information measuring apparatuses 1000 and 1000a described above in response to a user request for measuring biological information. The light-emitting and light-receiving pixel controllers 200 and 400 and/or the bio-signal processor 500 may each be implemented as an independent processor or integrated into the main processor ED21 or the auxiliary processor ED23 of the electronic device ED01.

The memory ED30 may store various types of data used by the components (e.g., the processor ED20, the sensor module ED76, etc.) of the electronic device ED01. The data may include, for example, software (e.g., program ED40, etc.) and input data and/or output data for commands related thereto. The memory ED30 may include the volatile memory ED32 and/or the nonvolatile memory ED34.

The program ED40 may be stored as software in the memory ED30, and may include an operating system ED42, middleware ED44, and/or an application ED46.

The input device ED50 may receive commands and/or data to be used for the components (e.g., the processor ED20, etc.) of the electronic device ED01 from the outside (e.g., a user, etc.) of the electronic device ED01. The input device ED50 may include a microphone, a mouse, a keyboard, and/or a digital pen (such as a stylus pen).

The sound output device ED55 may output a sound signal to the outside of the electronic device ED01. The sound output device ED55 may include a speaker and/or a receiver. The speaker may be used for general purposes, such as multimedia playback or recording playback, and the receiver may be used to receive incoming calls. The receiver may be combined as a part of the speaker or may be implemented as an independent separate device.

The display device ED60 may visually provide information to the outside of the electronic device ED01. The display device ED60 may include a display, a hologram device, or a projector, and a control circuit for controlling a corresponding device. The display device ED60 may include a touch circuitry set to sense a touch, and/or a sensor circuitry (a pressure sensor, etc.) set to measure the strength of a force generated by touch. The light-emitting pixel array 100 of FIGS. 1 and 6A may function as the display device ED60.

The audio module ED70 may convert sound into an electric signal or, conversely, convert an electric signal into sound. The audio module ED70 may acquire sound through the input device ED50 and may output sound through the sound output device ED55 and/or a speaker and/or headphones of another electronic device (an electronic device ED02, etc.) directly or wirelessly connected to the electronic device ED01.

The sensor module ED76 may detect the operation state (e.g., power, temperature, etc.) of the electronic device ED01 or the external environment state (e.g., user state, etc.) and may generate an electrical signal and/or data value corresponding to the detected state. The sensor module ED76 may include a gesture sensor, a gyro sensor, a barometric sensor, a magnetic sensor, an acceleration sensor, a grip sensor, a proximity sensor, a color sensor, an IR sensor, a biometric sensor, a temperature sensor, a humidity sensor, and/or an illuminance sensor. The biological information measuring apparatuses 1000 and 1000a described above with reference to FIGS. 1 and 6A may be one of the biometric sensors included in the sensor module ED76.

The interface ED77 may support one or more designated protocols that may be used to connect the electronic device ED01 directly or wirelessly to another electronic device (e.g., the electronic device ED02, etc.). The interface ED77 may include a High Definition Multimedia Interface (HDMI), a universal serial bus (USB) interface, an SD card interface, and/or an audio interface.

A connection terminal ED78 may include a connector through which the electronic device ED01 can be physically connected to another electronic device (e.g., the electronic device ED02, etc.). The connection terminal ED78 may include an HDMI connector, a USB connector, an SD card connector, and/or an audio connector (e.g., headphone connector, etc.).

The haptic module ED79 may convert an electrical signal into a mechanical stimulus (e.g., vibration, movement, etc.) or an electrical stimulus that a user can perceive through a tactile or motor sense. The haptic module ED79 may include a motor, a piezoelectric element, and/or an electrical stimulation device.

The camera module ED80 may captures still images and video. The camera module ED80 may include a lens assembly including one or more lenses, image sensors, image signal processors, and/or flashes. The lens assembly included in the camera module ED80 may collect light emitted from an object whose image is to be captured.

The power management module ED88 may manage power supplied to the electronic device ED01. The power management module ED88 may be implemented as a part of a power management integrated circuit (PMIC).

The battery ED89 may supply power to the components of the electronic device ED01. The battery ED89 may include a non-rechargeable primary cell, a rechargeable secondary cell, and/or a fuel cell.

The communication module ED90 may support establishment of a direct (wired) communication channel and/or a wireless communication channel between the electronic device ED01 and other electronic devices (e.g., the electronic device ED02, an electronic device ED04, a server ED08, etc.) in a network environment ED00m and communication through the established communication channel. The communication module ED90 may operate independently of the processor ED20 (e.g., an application processor, etc.) and may include one or more communication processors that supports direct communication and/or wireless communication. The communication module ED90 may include a wireless communication module ED92 (e.g., a cellular communication module, a short-range wireless communication module, a global navigation satellite system (GNSS) communication module, etc.) and/or a wired communication module ED94 (e.g., local area network (LAN) communication, a power line communication module, etc.). The communication module may communicate with another electronic device through a first network ED98 (e.g., a short-range communication network such as Bluetooth, Wi-Fi Direct, or infrared data association (IrDA)) or a second network (ED99) (e.g., a telecommunications network such as a cellular network, the Internet, or a computer network (e.g., LAN, WAN, etc.)) These various types of communication modules may be integrated into one component (e.g., a single chip, etc.), or may be implemented as a plurality of components (multiple chips) separate from each other. The wireless communication module ED92 may use subscriber information (e.g., international mobile subscriber identifier (IMSI), etc.) stored in the subscriber identification module ED96 to check and authenticate the electronic device ED01 in a communication network, such as such as the first network ED98 and/or the second network ED99.

The antenna module ED97 may transmit signals and/or power to the outside (such as other electronic devices) or receive from the outside. The antenna may include a radiator made of a conductive pattern formed on a substrate (e.g., PCB, etc.). The antenna module ED97 may include one or a plurality of antennas. In the case where a plurality of antennas are included, an antenna suitable for a communication method used in the communication network, such as the first network ED98 and/or the second network ED99, may be selected from among the plurality of antennas by the communication module ED90. Signals and/or power may be transmitted or received between the communication module ED90 and another electronic device through the selected antenna. In addition to the antenna, other components (such as RFIC) may be included as a part of the antenna module ED97.

Some of the components may be connected to each other through communication methods (e.g., a bus, a general purpose input and output (GPIO), a serial peripheral Interface (SPI), a mobile industry processor interface (MIPI), etc.) of peripheral devices and may exchange signals (e.g., commands, data, etc.) with each other.

The command or data may be transmitted or received between the electronic device ED01 and the external electronic device ED04 through the server ED08 connected to the second network ED99. The other electronic devices ED02 and ED04 may be the same as or different from the electronic device ED01 in types. All or some of the operations executed in the electronic device ED01 may be executed in one or more other electronic devices ED02, ED04, and ED08. For example, when the electronic device ED01 needs to perform a function or service, the electronic device ED01 may request one or more other electronic devices to perform part or all of the function or service instead of performing the function or service by itself. One or more other electronic devices that have received the request may perform an additional function or service related to the request, and transmit a result of the execution to the electronic device ED01. To this end, cloud computing, distributed computing, and/or client-server computing technology may be used.

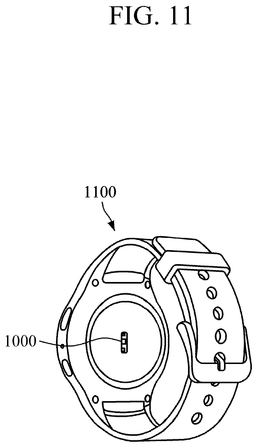

FIGS. 11 to 13 are diagrams illustrating an electronic device in which a biological information measuring apparatus is mounted.

Referring to FIG. 11, the electronic device ED01 of FIG. 10 may be implemented as a watch-type wearable device 1100, and may include a main body and a wrist strap. A display may be provided on the front of the main body to display various application screens showing time information and received message information. The biological information measuring apparatuses 1000 and 1000a may be disposed on the rear surface of the main body. The biological information measuring apparatus may output a light signal to a body region, such as a user's wrist, that is in contact with the rear surface of the main body, and measure a bio-signal by detecting light reflected from the body region. The electronic device 1100 may analyze the bio-signal measured by the biological information measuring apparatus to measure the user's biological information, such as a blood pressure, vascular age, arterial stiffness, aortic pressure waveform, stress index, and the like.

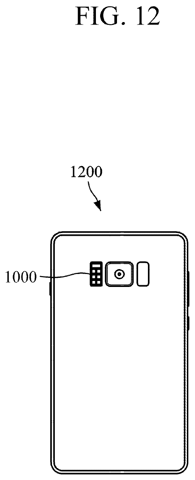

Referring to FIG. 12, the electronic device ED01 of FIG. 10 may be implemented as a mobile device 1200 such as a smart phone, and may include a housing and a display panel.

The housing may form the outer appearance of the mobile device 1200. The housing may include a first surface, a second surface opposite to the first surface, and a side surface surrounding the space between the first surface and the second surface. A display panel and a cover glass may be sequentially arranged on the first surface of the housing. The display panel may be exposed to the outside through the cover glass. On the second surface of the housing, a biological information measuring apparatus 1000 or 1000a, a camera module, and an infrared sensor may be disposed. When a user requests biological information by executing an application or the like mounted on the mobile device 1200, the biological information may be measured using the biological information measuring apparatus and the measured information may be provided as an image or audio to the user.

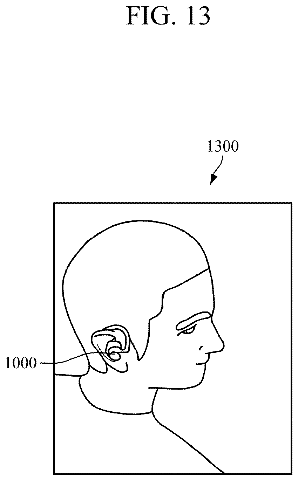

Referring to FIG. 13, the electronic device ED01 of FIG. 10 may be implemented as an ear wearable device 1300, and may include a main body and an ear strap.

The user may wear the electronic device 1300 of FIG. 13 by wearing the ear strap on the auricle. With the user wearing the electronic device 1300, the main body may be inserted into the external auditory meatus of the user. The main body may be equipped with a biological information measuring apparatus 1000 or 1000a. The biological information measuring apparatus may output a light signal to a body region that is in contact with the main body, such as a wall surface of a user's ear canal, and may measure a bio-signal by detecting light reflected from the body region. The electronic device 1300 of FIG. 13 may provide the biological information measured by the biological information measuring apparatus as sound to the user, or may transmit the biological information to an external device (e.g., mobile device, a tablet device, a PC, or the like) through a communication module equipped in the main body.

The scope of the invention is defined by the appended claims.

## Claims

1. A biological information measuring apparatus (1000) comprising:
a light-emitting pixel array (100) comprising a plurality of light-emitting pixel rows (110, 120, 130), wherein each of the light-emitting pixel rows comprises a plurality of light-emitting pixels (111, 112, 113, 114, 115, 116, 121, 122, 123, 124, 125, 126, 131, 132, 133, 134, 135, 136);
a light-receiving pixel array (300) comprising a plurality of light-receiving pixel rows (310, 320, 330), wherein each of the light-receiving pixel rows comprises a plurality of light-receiving pixels (311, 312, 313, 314, 315, 316, 321, 322, 323, 324, 325, 326, 331, 332, 333, 334, 335, 336), and wherein the light-receiving pixel array is configured to sample light that is emitted from the light-emitting pixel array and reflected by an object (OBJ); and
a bio-signal processor (500) configured to measure biological information using data sampled by the light-receiving pixel array,
wherein each of the light-emitting pixels comprises a storage capacitor (Cst) configured to store an nth light-emitting pixel signal and a memory capacitor (Cm) configured to store an (n+1)th light-emitting pixel signal,
**characterized in that**
the light-emitting pixel array comprises a refresh line (RL) for simultaneously updating pixel signals of at least two or more light-emitting pixels of the plurality of light-emitting pixels that are arranged in different light-emitting pixel rows (110, 120) of the plurality of light-emitting pixel rows, and
a refresh line signal applied to the light-emitting pixels through the refresh line (RL) changes the nth light-emitting pixel signal stored in the storage capacitor (Cst) to the (n+1)th light-emitting signal stored in the memory capacitor (Cm).

2. The biological information measuring apparatus (1000) of claim 1, wherein each of the light-emitting pixels (111, 112, 113, 114, 115, 116, 121, 122, 123, 124, 125, 126, 131, 132, 133, 134, 135, 136) comprises a refresh transistor (TRr) for transferring voltage charged in the memory capacitor to the storage capacitor.

3. The biological information measuring apparatus (1000) of claim 2, wherein the refresh transistor (TRr) has an end connected to the storage capacitor (Cst).

4. The biological information measuring apparatus (1000) of claim 3, wherein each of the light-emitting pixels (111, 112, 113, 114, 115, 116, 121, 122, 123, 124, 125, 126, 131, 132, 133, 134, 135, 136) comprises a transfer transistor (TRt) having a gate connected to the memory capacitor (Cm) and an end connected to the refresh transistor (TRr).

5. The biological information measuring apparatus (1000) of one of claims 1 to 4, wherein at least one of the plurality of light-emitting pixels (111, 112, 113, 114, 115, 116, 121, 122, 123, 124, 125, 126, 131, 132, 133, 134, 135, 136) and at least one of the plurality of light-receiving pixels (311, 312, 313, 314, 315, 316, 321, 322, 323, 324, 325, 326, 331, 332, 333, 334, 335, 336) share a driving voltage line (PL).

6. The biological information measuring apparatus (1000) of one of claims 1 to 5, wherein at least one of the plurality of light-emitting pixel rows (110, 120, 130) is interposed between the light-receiving pixel rows (310, 320, 330).

7. The biological information measuring apparatus (1000) of one of claims 1 to 6, wherein at least one of the plurality of light-receiving pixel rows (310, 320, 330) is interposed between the light-emitting pixel rows (110, 120, 130).

8. The biological information measuring apparatus (1000) of one of claims 1 to 7, further comprising a light-emitting pixel controller (200) configured to independently control each of the plurality of light-emitting pixels (111, 112, 113, 114, 115, 116, 121, 122, 123, 124, 125, 126, 131, 132, 133, 134, 135, 136).

9. The biological information measuring apparatus (1000) of one of claims 1 to 8, further comprising a light-receiving pixel controller (400) configured to drive the light-receiving pixel array (300) using a rolling shutter method or a global shutter method.

10. The biological information measuring apparatus (1000) of one of claims 1 to 9, wherein the light-emitting pixel array (100) comprises a first light-emitting pixel (111) configured to emit light of a first wavelength (IR) and a second light-emitting pixel (112) configured to emit light of a second wavelength (G) that is different from the first wavelength.

11. The biological information measuring apparatus (1000) of one of claims 1 to 10, wherein the biological information is photoplethysmogram, PPG, or blood pressure.

12. An electronic device (ED01; 1100; 1200; 1300) comprising:
a biological information measuring apparatus (1000) of one of claims 1 to 11;
a processor (ED20) configured to control an operation of the biological information measuring apparatus; and
a sound output device or a display device configured to output information measured by the biological information measuring apparatus.

13. A method of operating a biological information measuring apparatus (1000) comprising a light-emitting pixel array (100), a light-receiving pixel array (300), a light-emitting pixel controller (200), a light-receiving pixel controller (400), and a bio-signal processor (500), the method comprising the steps of:
emitting, by the light-emitting pixel array, light towards an object;
detecting, by the light-receiving pixel array, light reflected by the object;
driving, by the light-emitting pixel controller, the light-emitting pixel array;
driving, by the light-receiving pixel controller, the light-receiving pixel array; and
measuring, by the bio-signal processor, biological information based on signals detected by the light-receiving pixel array,
wherein the light-emitting pixel array comprises light-emitting pixels (111, 112, 113, 114, 115, 116, 121, 122, 123, 124, 125, 126, 131, 132, 133, 134, 135, 136), and wherein each
of the light-emitting pixels comprises a storage capacitor (Cst) configured to store an nth light-emitting pixel signal and the method further comprises storing, by a memory capacitor (Cm), an (n+1)th light-emitting pixel signal,
**characterized in that**
the light-emitting pixel array comprises a refresh line (RL) and the method further comprises simultaneously updating pixel signals of at least two or more light-emitting pixels of the light-emitting pixels that are arranged in different pixel rows (110, 120) of the light-emitting pixel array, and
a refresh line signal applied to the light-emitting pixels through the refresh line (RL) changes the nth light-emitting pixel signal stored in the storage capacitor (Cst) to the (n+1)th light-emitting signal stored in the memory capacitor (Cm).

## Patentansprüche

1. Vorrichtung (1000) zum Messen von biologischen Informationen, umfassend:
eine lichtemittierende Pixelmatrix (100), umfassend eine Vielzahl von lichtemittierenden Pixelreihen (110, 120, 130), wobei jede der lichtemittierenden Pixelreihen eine Vielzahl von lichtemittierenden Pixeln (111, 112, 113, 114, 115, 116, 121, 122, 123, 124, 125, 126, 131, 132, 133, 134, 135, 136) umfasst;
eine lichtempfangende Pixelmatrix (300), umfassend eine Vielzahl von lichtempfangenden Pixelreihen (310, 320, 330), wobei jede der lichtempfangenden Pixelreihen eine Vielzahl von lichtempfangenden Pixeln (311, 312, 313, 314, 315, 316, 321, 322, 323, 324, 325, 326, 331, 332, 333, 334, 335, 336) umfasst und wobei die lichtempfangende Pixelmatrix zum Abtasten von Licht ausgebildet ist, das von der lichtemittierenden Pixelmatrix ausgestrahlt und von einem Objekt (OBJ) reflektiert wird; und
einen Biosignalprozessor (500), ausgebildet zum Messen von biologischen Informationen unter Verwendung von Daten, die von der lichtempfangenden Pixelmatrix abgetastet werden,
wobei jedes der lichtemittierenden Pixel einen Speicherkondensator (Cst), der zum Speichern eines n-ten lichtemittierenden Pixelsignals ausgebildet ist, und einen Speicherkondensator (Cm), der zum Speichern eines (n+1)-ten lichtemittierenden Pixelsignals ausgebildet ist, umfasst,
**dadurch gekennzeichnet, dass**
die lichtemittierende Pixelmatrix eine Auffrischungsleitung (RL) zum gleichzeitigen Aktualisieren von Pixelsignalen von wenigstens zwei oder mehr lichtemittierenden Pixeln der Vielzahl von lichtemittierenden Pixeln umfasst, die in verschiedenen lichtemittierenden Pixelreihen (110, 120) der Vielzahl von lichtemittierenden Pixelreihen angeordnet sind, und
ein Auffrischungsleitungssignal, das durch die Auffrischungsleitung (RL) auf die lichtemittierenden Pixel angewendet wird, das n-te lichtemittierende Pixelsignal, das im Speicherkondensator (Cst) gespeichert ist, in das (n+1)-te lichtemittierende Signal, das im Speicherkondensator (Cm) gespeichert ist, ändert.

2. Vorrichtung (1000) zum Messen von biologischen Informationen nach Anspruch 1, wobei jedes der lichtemittierenden Pixel (111, 112, 113, 114, 115, 116, 121, 122, 123, 124, 125, 126, 131, 132, 133, 134, 135, 136) einen Auffrischungstransistor (TRr) zur Übertragung der im Speicherkondensator geladenen Spannung zum Speicherkondensator umfasst.

3. Vorrichtung (1000) zum Messen von biologischen Informationen nach Anspruch 2, wobei der Auffrischungstransistor (TRr) mit einem Ende mit dem Speicherkondensator (Cst) verbunden ist.

4. Vorrichtung (1000) zum Messen von biologischen Informationen nach Anspruch 3, wobei jedes der lichtemittierenden Pixel (111, 112, 113, 114, 115, 116, 121, 122, 123, 124, 125, 126, 131, 132, 133, 134, 135, 136) einen Übertragungstransistor (TRt) umfasst, von dem ein Gate mit dem Speicherkondensator (Cm) verbunden und ein Ende mit dem Auffrischungstransistor (TRr) verbunden ist.

5. Vorrichtung (1000) zum Messen von biologischen Informationen nach einem der Ansprüche 1 bis 4, wobei wenigstens eines der Vielzahl von lichtemittierenden Pixeln (111, 112, 113, 114, 115, 116, 121, 122, 123, 124, 125, 126, 131, 132, 133, 134, 135, 136) und wenigstens eines der Vielzahl von lichtempfangenden Pixeln (311, 312, 313, 314, 315, 316, 321, 322, 323, 324, 325, 326, 331, 332, 333, 334, 335, 336) eine Treiberspannungsleitung (PL) teilen.

6. Vorrichtung (1000) zum Messen von biologischen Informationen nach einem der Ansprüche 1 bis 5, wobei wenigstens eine der Vielzahl von lichtemittierenden Pixelreihen (110, 120, 130) zwischen den lichtempfangenden Pixelreihen (310, 320, 330) angeordnet ist.

7. Vorrichtung (1000) zum Messen von biologischen Informationen nach einem der Ansprüche 1 bis 6, wobei wenigstens eine der Vielzahl von lichtempfangenden Pixelreihen (310, 320, 330) zwischen den lichtemittierenden Pixelreihen (110, 120, 130) angeordnet ist.

8. Vorrichtung (1000) zum Messen von biologischen Informationen nach einem der Ansprüche 1 bis 7, ferner umfassend ein Steuergerät (200) für lichtemittierende Pixel, ausgebildet zum unabhängigen Steuern von jedem der Vielzahl von lichtemittierenden Pixeln (111, 112, 113, 114, 115, 116, 121, 122, 123, 124, 125, 126, 131, 132, 133, 134, 135, 136).

9. Vorrichtung (1000) zum Messen von biologischen Informationen nach einem der Ansprüche 1 bis 8, ferner umfassend ein Steuergerät (400) für lichtempfangende Pixel, ausgebildet zum Antreiben der lichtempfangenden Pixelmatrix (300) unter Verwendung eines Rolling-Shutter-Verfahrens oder eines Global-Shutter-Verfahrens.

10. Vorrichtung (1000) zum Messen von biologischen Informationen nach einem der Ansprüche 1 bis 9, wobei
die lichtemittierende Pixelmatrix (100) ein erstes lichtemittierendes Pixel (111), ausgebildet zum Ausstrahlen von Licht einer ersten Wellenlänge (IR), und ein zweites lichtemittierendes Pixel (112), ausgebildet zum Ausstrahlen von Licht einer zweiten Wellenlänge (G), die sich von der ersten Wellenlänge unterscheidet, umfasst.

11. Vorrichtung (1000) zum Messen von biologischen Informationen nach einem der Ansprüche 1 bis 10, wobei die biologischen Informationen ein Photoplethysmogramm, ein PPG oder ein Blutdruck sind.

12. Elektronische Vorrichtung (ED01; 1100; 1200; 1300), umfassend:
eine Vorrichtung (1000) zum Messen von biologischen Informationen nach einem der Ansprüche 1 bis 11;
einen Prozessor (ED20), ausgebildet zum Steuern eines Betriebs der Vorrichtung zum Messen von biologischen Informationen; und
eine Audioausgabevorrichtung oder eine Anzeigevorrichtung, ausgebildet zum Ausgeben von Informationen, die von der Vorrichtung zum Messen von biologischen Informationen gemessen werden.

13. Verfahren zum Betreiben einer Vorrichtung (1000) zum Messen von biologischen Informationen, umfassend eine lichtemittierende Pixelmatrix (100), eine lichtempfangende Pixelmatrix (300), ein Steuergerät (200) für lichtemittierende Pixel, ein Steuergerät (400) für lichtempfangende Pixel und einen Biosignalprozessor (500), wobei das Verfahren die Schritte umfasst zum:
Ausstrahlen von Licht in Richtung eines Objekts durch die lichtemittierende Pixelmatrix; Erfassen von durch das Objekt reflektiertem Licht durch die lichtempfangende Lichtmatrix; Antreiben der lichtemittierenden Pixelmatrix durch das Steuergerät für lichtemittierende Pixel; Antreiben der lichtempfangenden Pixelmatrix durch das Steuergerät für lichtempfangende Pixel; Messen von biologischen Informationen durch den Biosignalprozessor auf der Basis von durch die lichtempfangende Pixelmatrix erfassten Signalen,
wobei die lichtemittierende Pixelmatrix lichtemittierende Pixel (111, 112, 113, 114, 115, 116, 121, 122, 123, 124, 125, 126, 131, 132, 133, 134, 135, 136) umfasst und wobei jedes der lichtemittierenden Pixel einen Speicherkondensator (Cst) umfasst, ausgebildet zum Speichern eines n-ten lichtemittierenden Pixelsignals, und das Verfahren ferner das Speichern eines (n+1)-ten lichtemittierenden Pixelsignals durch einen Speicherkondensator (Cm) umfasst, **dadurch gekennzeichnet, dass**
die lichtemittierende Pixelmatrix eine Auffrischungsleitung (RL) umfasst und das Verfahren ferner das gleichzeitige Aktualisieren von Pixelsignalen von wenigstens zwei oder mehr lichtemittierenden Pixeln der lichtemittierenden Pixel umfasst, die in verschiedenen Pixelreihen (110, 120) der lichtemittierenden Pixelmatrix angeordnet sind, und
ein Auffrischungsleitungssignal, das durch die Auffrischungsleitung (RL) auf die lichtemittierenden Pixel angewendet wird, das n-te lichtemittierende Pixelsignal, das im Speicherkondensator (Cst) gespeichert ist, in das (n+1)-te lichtemittierende Signal, das im Speicherkondensator (Cm) gespeichert ist, ändert.

## Revendications

1. Appareil de mesure d'informations biologiques (1000) comprenant :
un réseau de pixels émetteurs de lumière (100) comprenant une pluralité de rangées de pixels émetteurs de lumière (110, 120, 130), dans lequel chacune des rangées de pixels émetteurs de lumière comprend une pluralité de pixels émetteurs de lumière (111, 112, 113, 114, 115, 116, 121, 122, 123, 124, 125, 126, 131, 132, 133, 134, 135, 136) ;
un réseau de pixels récepteurs de lumière (300) comprenant une pluralité de rangées de pixels récepteurs de lumière (310, 320, 330), dans lequel chacune des rangées de pixels récepteurs de lumière comprend une pluralité de pixels récepteurs de lumière (311, 312, 313, 314, 315, 316, 321, 322, 323, 324, 325, 326, 331, 332, 333, 334, 335, 336), et dans lequel le réseau de pixels récepteurs de lumière est configuré pour échantillonner de la lumière qui est émise par le réseau de pixels émetteurs de lumière et réfléchie par un objet (OBJ) ; et
un processeur de bio-signal (500) configuré pour mesurer des informations biologiques en utilisant des données échantillonnées par le réseau de pixels récepteurs de lumière, dans lequel chacun des pixels émetteurs de lumière comprend un condensateur de stockage (Cst) configuré pour stocker un n-ième signal de pixel émetteur de lumière et un condensateur mémoire (Cm) configuré pour stocker un (n+1)-ième signal de pixel émetteur de lumière,
caractérisé en ce
le réseau de pixels émetteurs de lumière comprend une ligne de rafraîchissement (RL) pour mettre à jour simultanément des signaux de pixel d'au moins deux pixels émetteurs de lumière ou plus de la pluralité de pixels émetteurs de lumière qui sont disposés dans différentes rangées de pixels émetteurs de lumière (110, 120) de la pluralité des rangées de pixels émetteurs de lumière, et
un signal de ligne de rafraîchissement appliqué aux pixels émetteurs de lumière via la ligne de rafraîchissement (RL) transforme le n-ième signal de pixel émetteur de lumière stocké dans le condensateur de stockage (Cst) en le (n+1)-ième signal émetteur de lumière stocké dans le condensateur mémoire (Cm).

2. Appareil de mesure d'informations biologiques (1000) selon la revendication 1, dans lequel chacun des pixels émetteurs de lumière (111, 112, 113, 114, 115, 116, 121, 122, 123, 124, 125, 126, 131, 132, 133, 134, 135, 136) comprend un transistor de rafraîchissement (TRr) pour transférer une tension chargée dans le condensateur mémoire vers le condensateur de stockage.

3. Appareil de mesure d'informations biologiques (1000) selon la revendication 2, dans lequel le transistor de rafraîchissement (TRr) a une extrémité connectée au condensateur de stockage (Cst).

4. Appareil de mesure d'informations biologiques (1000) selon la revendication 3, dans lequel chacun des pixels émetteurs de lumière (111, 112, 113, 114, 115, 116, 121, 122, 123, 124, 125, 126, 131, 132, 133, 134, 135, 136) comprend un transistor de transfert (TRt) ayant une grille connectée au condensateur mémoire (Cm) et une extrémité connectée au transistor de rafraîchissement (TRr).

5. Appareil de mesure d'informations biologiques (1000) selon l'une des revendications 1 à 4, dans lequel au moins un de la pluralité de pixels émetteurs de lumière (111, 112, 113, 114, 115, 116, 121, 122, 123, 124, 125, 126, 131, 132, 133, 134, 135, 136) et au moins un de la pluralité de pixels récepteurs de lumière (311, 312, 313, 314, 315, 316, 321, 322, 323, 324, 325, 326, 331, 332, 333, 334, 335, 336) partagent une ligne de tension d'attaque (PL).

6. Appareil de mesure d'informations biologiques (1000) selon l'une des revendications 1 à 5, dans lequel au moins une de la pluralité de rangées de pixels émetteurs de lumière (110, 120, 130) est intercalée entre les rangées de pixels récepteurs de lumière (310, 320, 330).

7. Appareil de mesure d'informations biologiques (1000) selon l'une des revendications 1 à 6, dans lequel au moins une de la pluralité de rangées de pixels récepteurs de lumière (310, 320, 330) est intercalée entre les rangées de pixels émetteurs de lumière (110, 120, 130).

8. Appareil de mesure d'informations biologiques (1000) selon l'une des revendications 1 à 7, comprenant en outre un dispositif de commande de pixels émetteurs de lumière (200) configuré pour commander indépendamment chacun de la pluralité de pixels émetteurs de lumière (111, 112, 113, 114, 115, 116, 121, 122, 123, 124, 125, 126, 131, 132, 133, 134, 135, 136).

9. Appareil de mesure d'informations biologiques (1000) selon l'une des revendications 1 à 8, comprenant en outre un dispositif de commande de pixels récepteurs de lumière (400) configuré pour attaquer le réseau de pixels récepteurs de lumière (300) en utilisant un procédé à obturateur roulant ou un procédé à obturateur global.

10. Appareil de mesure d'informations biologiques (1000) selon l'une des revendications 1 à 9, dans lequel le réseau de pixels émetteurs de lumière (100) comprend un premier pixel émetteur de lumière (111) configuré pour émettre une lumière d'une première longueur d'onde (IR) et un second pixel émetteur de lumière (112) configuré pour émettre une lumière d'une seconde longueur d'onde (G) qui est différente de la première longueur d'onde.

11. Appareil de mesure d'informations biologiques (1000) selon l'une des revendications 1 à 10, dans lequel les informations biologiques sont un photopléthysmogramme, PPG, ou une pression artérielle.

12. Dispositif électronique (ED01 ; 1100 ; 1200 ; 1300) comprenant :
un appareil de mesure d'informations biologiques (1000) selon l'une des revendications 1 à 11 ;
un processeur (ED20) configuré pour commander un fonctionnement de l'appareil de mesure d'informations biologiques ; et
un dispositif de sortie sonore ou un dispositif d'affichage configuré pour délivrer en sortie des informations mesurées par l'appareil de mesure d'informations biologiques.

13. Procédé de fonctionnement d'un appareil de mesure d'informations biologiques (1000) comprenant un réseau de pixels émetteurs de lumière (100), un réseau de pixels récepteurs de lumière (300), un dispositif de commande de pixels émetteurs de lumière (200), un dispositif de commande de pixels récepteurs de lumière (400) et un processeur de bio-signal (500), le procédé comprenant les étapes consistant à :
émettre, par le réseau de pixels émetteurs de lumière, de la lumière vers un objet ;
détecter, par le réseau de pixels récepteurs de la lumière, de la lumière réfléchie par l'objet ;
attaquer, par le dispositif de commande de pixels émetteurs de lumière, le réseau de pixels émetteurs de lumière ;
attaquer, par le dispositif de commande de pixels récepteurs de lumière, le réseau de pixels récepteurs de lumière ; et
mesurer, par le processeur de bio-signal, des informations biologiques basées sur des signaux détectés par le réseau de pixels récepteurs de la lumière,
dans lequel le réseau de pixels émetteurs de lumière comprend des pixels émetteurs de lumière (111, 112, 113, 114, 115, 116, 121, 122, 123, 124, 125, 126, 131, 132, 133, 134, 135, 136), et dans lequel chacun des pixels émetteurs de lumière comprend un condensateur de stockage (Cst) configuré pour stocker un n-ième signal de pixel émetteur de lumière et le procédé comprend en outre le stockage, par un condensateur mémoire (Cm), d'un (n+1)-ième signal de pixel émetteur de lumière,
caractérisé en ce
le réseau de pixels émetteurs de lumière comprend une ligne de rafraîchissement (RL) et le procédé comprend en outre la mise à jour simultanée de signaux de pixel d'au moins deux pixels émetteurs de lumière ou plus des pixel émetteurs de lumière qui sont disposés dans différentes rangées de pixels (110, 120) du réseau de pixels émetteurs de lumière, et
un signal de ligne de rafraîchissement appliqué aux pixels émetteurs de lumière via la ligne de rafraîchissement (RL) transforme le n-ième signal de pixel émetteur de lumière stocké dans le condensateur de stockage (Cst) en le (n+1)-ième signal émetteur de lumière stocké dans le condensateur mémoire (Cm).
